# EUROPEAN PATENT SPECIFICATION

(11) **EP 3 735 173 B1**
(45) Date of publication and mention of the grant of the patent: **05.07.2023**
(21) Application number: 18898107.0
(22) Date of filing: 21.12.2018
(51) Int. Cl.: A61B 5/389, A41D 13/005, A61B 5/053, A61B 5/107, A61F 7/00, A61N 1/36, H05B 1/02

(54) **MULTI-FUNCTIONAL TUBULAR WORN GARMENT**
MULTIFUNKTIONELLES SCHLAUCHFÖRMIGES GETRAGENES KLEIDUNGSSTÜCK
VÊTEMENT PORTÉ TUBULAIRE, MULTIFONCTIONNEL

(30) Priority: 05.01.2018 US 201862614304 P
(43) Date of publication of application: 11.11.2020
(73) Proprietor: Myant Inc., Toronto, ON M5V 0A1 (CA)
(72) Inventor: CHAHINE, Tony, Toronto, Ontario M5V 0A1 (CA); AITKEN, Steve, Toronto, Ontario M5V 0A1 (CA); STRAKA, Adrian, Toronto, Ontario M5V 0A1 (CA); ALIZADEH-MEGHRAZI, Milad, Toronto, Ontario M5V 0A1 (CA)
(74) Representative: Hernandez Lehmann, Aurelio
(86) International application number: PCT/CA2018/051656
(87) International publication number: WO 2019/134033

(56) References cited:
- WO-A1-2015/138515
- WO-A2-2016/128778
- RU-A- 2009 138 322
- RU-C2- 2 437 687
- US-A- 5 032 705
- US-A1- 2014 318 699
- US-A1- 2014 353 300
- US-A1- 2015 088 043
- US-A1- 2015 366 504
- US-A1- 2016 135 251
- US-A1- 2017 036 066
- US-B2- 9 582 072
- US-B2- 10 119 208
- MURAI AKITO ET AL: "Evaluation of lower leg swelling using EMG measured with voltage divider", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 3751-3754, XP032674962, DOI: 10.1109/EMBC.2014.6944439 [retrieved on 2014-11-02]
- PETERS E J G ET AL: "The benefit of electrical stimulation to enhance perfusion in persons with diabetes mellitus", JOURNAL OF FOOT AND ANKLE SURGERY, WILLIAMS AND WILKINS, BALTIMORE, MD, US, vol. 37, no. 5, 1 September 1998 (1998-09-01), pages 396-400, XP025985069, ISSN: 1067-2516, DOI: 10.1016/S1067-2516(98)80048-3 [retrieved on 1998-09-01]

## Description

### CROSS REFERENCE TO RELATED APPLICATIONS

This application claims priority from U.S. provisional patent application No. 62/614,304, filed on January 5, 2018.

### TECHNICAL FIELD

The present invention relates in general to wearable electronics and smart textiles.

### BACKGROUND

Smart textiles are materials that sense and react to environmental conditions or stimuli, such as those from mechanical, thermal, chemical, electrical, magnetic or other sources. Smart textiles are materials that can react or adapt to external stimuli or changing environmental conditions. The stimuli can include changes in temperature, moisture, pH, chemical sources, electric or magnetic fields, mechanical stress or strain. Advanced smart textiles can have embedded computing, digital components, electronics, energy supply, and sensors. Basic components of a smart textile system include: sensors, actuators, data transmission and electrical power. Due to the discrete nature, size and comfort, a tubular shaped garment, such as a sock, knee brace, elbow sleeve, stocking, legging and the like are especially attractive form factors for a smart textile in particular for applications involving health & wellness and performance sports, where a sock can be used to detect and monitor a wide range of health issues, including: tracking of gait, pressure sensing, electromyography (EMG), heat stimulation and electrical muscle stimulation (EMS) of the calf for improved circulation and bio-impedance feedback for sub-skin infection monitoring and other combined features.

Current issues in the field of smart textiles relate to difficulties in application to therapy for diseases and other medical conditions. The appropriate configuration and application of on-textile sensor arrangements is problematic given today's available solutions. Further, the ability to properly regulate temperature of resistive heating in variable stretch environments is desired. A garment including EMS sensors is known from US 9 582 072 B2.

### SUMMARY

It is an object of the present invention to provide a tubular garment to obviate or mitigate at least one of the above presented disadvantages. A tubular garment according to the invention is defined in claim 1.

### BRIEF DESCRIPTION OF THE DRAWINGS

The non-limiting embodiments may be more fully appreciated by reference to the following detailed description of the non-limiting embodiments when taken in conjunction with the accompanying drawings, by example only, in which:
Figure 1 provides example front perspective and rear views of a tubular garment;
Figure 2 is an example operation of the sensor platform of the garment of Figure 1;
Figure 3 is a further embodiment of the garment of Figure 1 as a perspective front view;
Figure 4 a further example operation of the garment of Figure 3;
Figure 5 provides a further example embodiment as front perspective and rear views of the tubular garment of Figure 1;
Figure 6 provides a still further example embodiment as front perspective and rear views of the tubular garment of Figure 1;
Figure 7 is an example operation of the sensor platform of the garment of Figure 3;
Figure 8 shows an example construction of the tubular garment of Figure 1;
Figure 9 shows a example types of the tubular garment of Figure 1;
Figure 10 shows an example controller device of the tubular garment of Figure 1;
Figure 11 shows an example setup for a pair of tubular garments of Figure 1; and
Figure 12 shows an example operation of the tubular garment of Figure 6.

### DEFINITIONS

EMG: Electromyography. Measurement of the electrical signals generated by muscles.

EMS: Electromyostimulation. Providing external electrical energy to a muscle for stimulation.

IMU: Inertial Measurement Unit. A device that reports a body's force, acceleration and/or tilt (angle).

Bio-Impedance: the electrical resistance of a living organism in response to an externally applied electric current.

### DETAILED DESCRIPTION

These embodiments are described in sufficient detail to enable those skilled in the art to practice the invention, but other embodiments may be utilized and logical, mechanical, electrical, and other changes may be made without departing from the scope of the present invention. The following detailed description is, therefore, not to be taken in a limiting sense, and the scope of the present invention is defined only by the appended claims.

In the following description, specific details are set forth to provide a thorough understanding of the invention. However, it is understood that the invention may be practiced without these specific details. In other instances, well-known structures and techniques known to one of ordinary skill in the art have not been shown in detail in order not to obscure the invention. Referring to the figure, it is possible to see the various major elements constituting the apparatus of the present invention.

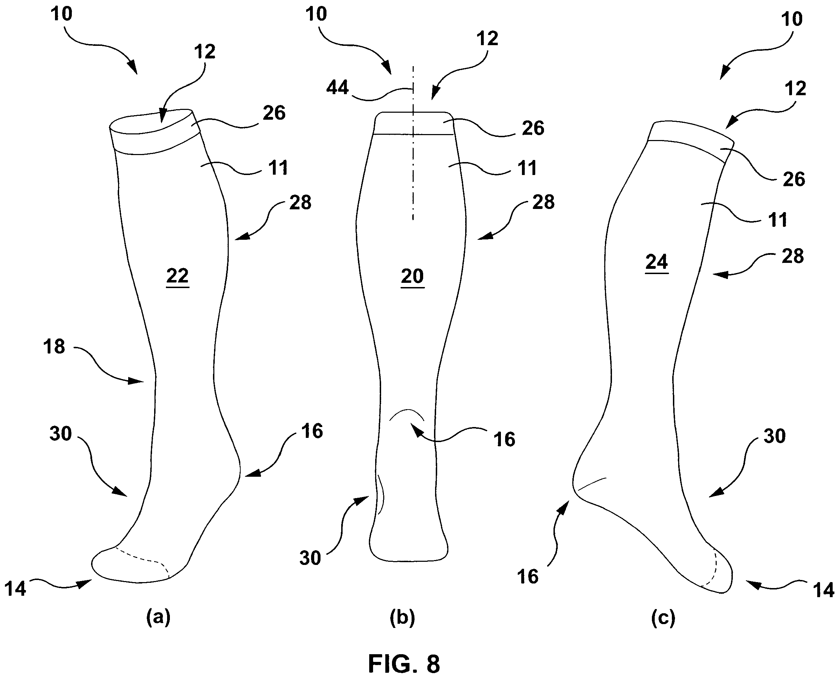

Referring to Figures 8a,b,c, shown is a tubular garment 10 (e.g. a sock) having a first open end 12 with an optional band 26 and a second end 14 (e.g. closed as shown or open as shown in ghost view). In terms of a sock 10 worn on a foot of a wearer, the second end 14 is positioned in the region of wearer's toes and the first open end 12 is positioned between the wearer's calf muscle and the wearer's knee. The garment 10 has an intermediate region 16 (e.g. ankle region or other joint region) separating a top portion 28 from a bottom portion 30. In terms of a sock 10, the top portion 28 is positioned about the wearer's calf and the bottom portion 30 is positioned about the wearer's foot, whereby the intermediate portion is positioned about the ankle. In general, the bottom portion 30 is for positioning towards a limb extremity of the wearer (e.g. forearm region of an arm, shin region of a leg, foot region of a leg, hand region of an arm) that is adjacent the respective joint (e.g. elbow, knee, ankle, wrist). Further, the top portion 28 is for positioning opposite to the limb extremity of the wearer (e.g. bicep region of an arm, thigh region of a leg, calf region of a leg, and forearm region of an arm) that is also adjacent to the respective joint between the portions 28, 30. For purposes of demonstration only, the tubular garment 10 is described in relation to being worn on (i.e. covering) a wearer's foot, ankle and calf body parts. However, it is also recognized that the tubular garment can be worn about the wrist (i.e. covering the hand, wrist and forearm), about the elbow (i.e. covering the forearm, elbow and bicep), or about the knee (i.e. covering the shin, knee, and thigh).

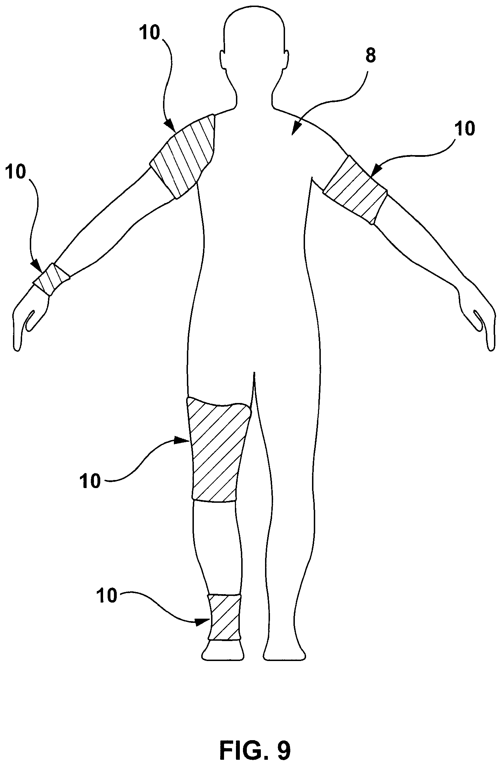

The garment 10, e.g. a textile-based product, can be used by a user/wearer 8 (such as, a human) - see Figure 9. The garment 10 includes (and is not limited to) any one of a knitted textile, a woven textile, or a cut and sewn textile, a knitted fabric, a non-knitted fabric, in any combination and/or permutation thereof (any equivalent thereof). The garment 10 can include an integrated functional textile article. It will be appreciated that some embodiments described a knitted garment, and it is understood that these embodiments may be extended to any textile fabric forms and/or techniques such as (weaving, knitting - warp, weft etc.), and the embodiments are not limited to a knitted garment. It will be appreciated that (where indicated) the FIGS (drawings) may be directed to a knitted garment body layer 11, and it will be appreciated that the knitted garment body layer 11 is an example of any form of textile fabrics forms and techniques such as (weaving, knitting - warp, weft etc.) for the garment body layer 11, and that any description and/or illustration to the knitted garment fabric does this limit the scope of the present embodiments. In accordance with an embodiment, there is provided a garment 10 made with any textile forming technique (and the knitted fabric garment is simply an example of such an arrangement).

Referring again to Figures 8a, b, c, the tubular garment 10 has a body 11 includes a front region 18, a rear region 20, a first side 22 between the regions 18, 20 and a second side 24 opposite the first side 22 and also between the regions 18,20. Thus the regions 18, 20 and sides 22, 24 form the tubular configuration of the body 11 of the garment 10. The body 11 is constructed out of a plurality of fibres, knit and/or woven, as further descried below.

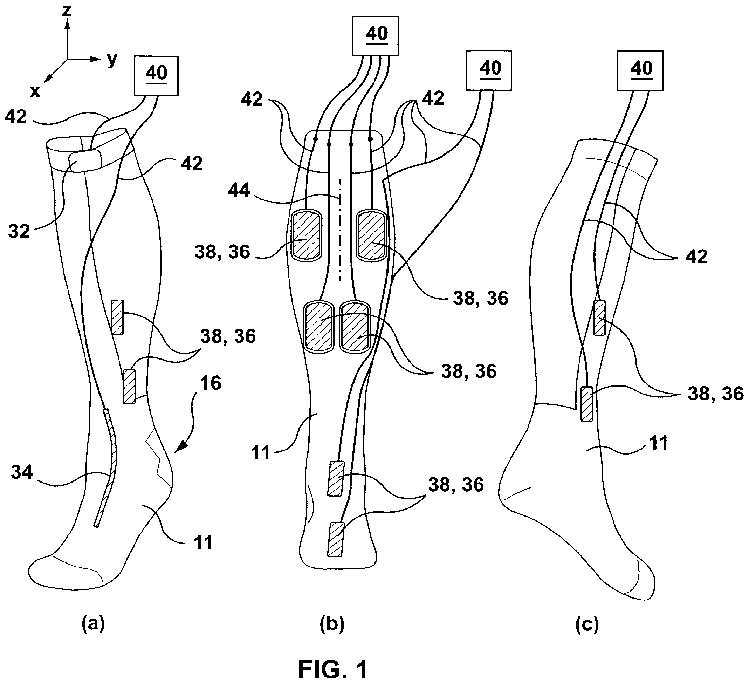

Referring to Figure 1, the garment 10 (shown only by example as a sock) can have an inertial measurement unit (IMU) sensor 32 connected to the body 11 (e.g. about the band 26) as an electronic device (e.g. sensor) that measures and reports a body's (e.g. limb of the wearer) specific force, angular rate, and/or sometimes the magnetic field surrounding the body, using a combination of accelerometers and gyroscopes, sometimes also magnetometers. Example configurations of the IMU sensor 32 can be used to detect linear acceleration of the wearer's limb using one or more onboard accelerometers and rotational rate using one or more onboard gyroscopes. Some IMU sensor 32 can also include an onboard magnetometer used as a heading reference. Typical configurations of the IMU sensor 32 contain one accelerometer, gyro, and magnetometer per axis for each of the three axes: x, y and z.

The garment 10 has one or more stretch/strain sensors 34 positioned on/in the body 11 and across the intermediate region 16 (e.g. extending from the top portion 28, across the intermediate region 16 and to the bottom portion 30) in order to detect flexure of the wearer's joint underlying the intermediate region 16, as the wearer moves the limb during physical activity (e.g. walking, running, lifting, carrying, or otherwise engaging relative movement of the limb with respect to the rest of the wearer's body). The top portion 28 and the bottom portion 30 can be oriented at an angle to one another about the intermediate region 16. For example, the stretch/strain sensors 34 can be applied to a surface of the body 11 material (e.g. consisting of nonconductive interlaced fibres). Alternatively, the stretch/strain sensors 34 can be composed of conductive fibres that are interlaced (e.g. knit or woven) with the fibres of the body 11 material. As further described below, other sensors can be provides, such as but not limited to temperature sensors/actuators 39 and pressure sensors/actuators 41.

The sensor 34,36,38,39,41,43 can be one or more conductive threads/fibres woven or knit into a pattern at specified locations of the garment 10 in the garment body layer 11 as part of the plurality of fibres thereof. The pattern of conductive threads (i.e. sensor 34,36,38,39,41,43) can form one or more circuits (e.g. bridge circuit) and electricity supplied to the pattern of conductive threads (e.g. from a power source attached to the suit) can be measured in the circuit to detect changes in capacitance and/or resistance of the thread pattern as the garment 10 fabric adjacent the conductive thread pattern is stretched. For example, the thread pattern (i.e. sensor 34) can be stretched along with the garment 10 as the garment wearer 8 tenses muscles adjacent the garment 10 in the vicinity of the thread patterns 34. The pattern of conductive thread (i.e. sensor 34) can be any pattern and can include aesthetic aspects including one or more colours which are visible on the background colour(s) of fabric adjacent the conductive thread pattern 34.

The electrically conductive thread incorporated into the garment 10 as one or more sensors 34,36,38,39,41,43 can be made of any conductive material including conductive metals such as stainless steel, silver, aluminium, copper, etc. In one embodiment, the conductive thread can be insulated. In another embodiment, the conductive thread can be uninsulated. Typically the electrically conductive thread is inter-knit or woven with other textile-based threads (i.e. non-conductive or insulating) making up the body 11 of the garment 10. The other textile-based threads making up the body 11 of the garment 10 can include any textile material such as cotton, spandex, nylon, polyester, and/or various synthetic materials. The electrically conductive thread incorporated into the garment 10 as one or more conductive pathways 42 can be made of any conductive material including conductive metals such as stainless steel, silver, aluminium, copper, etc. In one embodiment, the conductive thread can be insulated. In another embodiment, the conductive thread can be uninsulated. Typically the electrically conductive thread is inter-knit or woven with other textile-based threads (i.e. non-conductive or insulating) making up the body 11 of the garment 10. The other textile-based threads making up the body 11 of the garment 10 can include any textile material such as cotton, spandex, nylon, polyester, and/or various synthetic materials.

Capacitance and/or resistance can be measured across all or a portion of conductive thread and/or pattern of conductive thread. For example, changes in resistance and/or capacitance of the conductive thread can be measured using a bridge circuit (e.g. a Wheatstone bridge or Wien bridge) contained or otherwise sensed by the controller device 40, a type of electrical circuit in which two circuit branches are "bridged" by a third branch connected between the first two branches at some intermediate point along them. A source of power (e.g. a battery) of the controller device 40 can be connected to the bridge circuit along with a measuring device (e.g. a voltmeter, ammeter, or galvanometer) of the controller device 40 to detect changes in the resistance or capacitance of the conductive thread (i.e. sensor 34) as the thread changes length/width/thickness or other shape (e.g. due to stretching of the thread in response to tension in muscles adjacent to the thread). Therefore the circuit can be calibrated to measure changes in length/width/thickness or other shape of the sensors 34 reflected as changes in the resistance and/or capacitance of the sensors 34.

It will be understood that the stretch sensor 34 (e.g. conductive thread) when attached/integrated to/into the fabric body layer 11 of a garment 10 can stretch when a skin surface underlying the stretch sensor 34 moves and/or stretches (e.g. as a result of the activation of a muscle or muscle group controlling movement of the skin surface). Stretching of the stretch sensor 34 can result in generation of signals that can be communicated (e.g. via a cord or wires) to a receiving device 40 (e.g. an electronic device attached to the garment 10). For example, the stretch sensor 34 can be configured to generate an electric signal in response to stretching/elongation (i.e. the stretch sensor 34 can self-report on changes to its length). In another embodiment, an electric circuit (e.g. bridge circuit) can be attached to the stretch sensor 34 (e.g. conductive thread) for measuring changes in capacitance or resistance across the sensor 34 as the sensor changes in shape (i.e. an electric circuit can report on changes detected in resistance and/or capacitance of the stretch sensor 34). The electric circuit can include a measuring device (e.g. ammeter, voltmeter, galvanometer) which can measure changes in the resistance and/or capacitance of the stretch sensor 34 and report the measured changes to an electronic device 40 of the garment 10 for processing (e.g. via a processor of the device 40).

The garment 10 also has Electromyography (EMG) sensors 36 on/in the body 11 used for evaluating and recording/detecting electrical activity produced by skeletal muscles (e.g. calf muscles, forearm muscles, bicep/tricep muscles, hand muscles, and general foot/leg muscles such as but not limited to dorsiflexor and plantarflexor muscles). EMG sensors 36 can be used to detect/record the electric potential generated by muscle cells when these cells are electrically or neurologically activated (e.g. by the wearer's brain in order to effect movement of the limb). The EMG signals detected by the EMG sensors 36 can be analyzed to detect medical abnormalities, activation level, or recruitment order, or to analyze the biomechanics of human or animal movement. For example, the EMG sensors 36 can be applied to a surface of the body 11 material (e.g. consisting of nonconductive interlaced fibres). Alternatively, the EMG sensors 36 can be composed of conductive fibres that are interlaced (e.g. knit or woven) with the fibres of the body 11 material.

The garment 10 also has Electrical muscle stimulation (EMS) actuators 38, also known as neuromuscular electrical stimulation (NMES) or electromyostimulation, which is the elicitation of muscle contraction using electric impulses applied by the EMS actuators 38. The impulses are transmitted to the EMS actuators 38 and delivered through the electrodes (i.e. the EMS actuators 38) on the wearer's skin near to the muscles being stimulated. The EMS actuators 38 can be pads that are positioned or otherwise biased into engagement with the skin. For example, the nonconductive fibres of the body 11 material can be resilient (e.g. elastic) in nature and thus promote contact of the sensors 36,38 with the skin of the wearer underlying the body 11 of the garment 10. As such, the EMS impulses applied by the EMS actuators 38 can mimic the action potential that comes from the central nervous system, causing the underlying muscles to contract and thus promote movement of the underlying skeletal structure of the limb. For example, the EMS actuators 38 can be applied to a surface of the body 11 material (e.g. consisting of nonconductive interlaced fibres). Alternatively, the EMS actuators 38 can be composed of conductive fibres that are interlaced (e.g. knit or woven) with the fibres of the body 11 material. It is recognized that the EMS actuators 38 and the EMG sensors 36 can be the same, or different, electrical components connected to a control unit 40 via a series of conductive pathways 42.

Referring again to Figures 1 and 8a, b, c, the sensors 36,38 (and/or sensors 34,39,41,43) can be positioned in a plurality of locations of the body 11, in particular adjacent to where the target wearer's muscles would be located upon wearing of the garment 10. For example, the sensors 36, 38 can be located on the first side 22 and the second side 24 on the top portion 28 (near the wearer's shin) adjacent to the intermediate region 16 (e.g. the ankle). For example, the sensors 36, 38 can be located on the rear region 20 between the first open end 12 and the intermediate region 16 on the top portion 28 (e.g. the calf muscle and/or plantar flexor region of the leg) adjacent to the intermediate region 16 (e.g. the ankle). For example, the sensors 36, 38 can be located on the rear region 20 of the bottom portion 30 (e.g. the sole of the foot) between the second end 12 and the intermediate region 16.

In terms of a sock as the tubular garment 10, the sensors 36, 38 on the rear region 20 of the bottom portion 30 can be positioned as a pair of sensors 36, 38, one towards the intermediate region 16 and one towards the second end 14, such that the pair of sensors 36,38 are spaced apart from one another. The sensors 36, 38 located on the first side 22 and the second side 24 on the top portion 28 can be each positioned as a pair of sensors 36, 38, one adjacent to the intermediate region 16 and one between the intermediate region 16 and the first open end 12, such that the pair of sensors 36,38 are spaced apart from one another. For example, the sensors 36, 38 located on the rear region 20 between the first open end 12 and the intermediate region 16 on the top portion 28 can be positioned as one or more pairs of sensors 36, 38, such that one of the pair is located towards the intermediate region and the other of the pair is located towards the first open end 12, such that the pair of sensors 36, 38 are spaced apart from one another. In terms of multiple pairs of sensors 36, 38 located on the rear region 20 between the first open end 12 and the intermediate region 16 on the top portion 28, each of the multiple pairs of sensors 36, 38 can be located to one side of a centerline 44 of the body 11 dividing the first side 22 from the second side 24. The additional EMS actuators 38 on the sole of the garment 10 can be utilized for the plantar and heel region.

In general terms, the sensors 36, 38 (also which can be referred to interchangeably as actuators 36, 38 depending upon whether the sensor/actuator is generating or receiving an electrical signal with respect to muscle activity) can be associated with detecting (e.g. electrical signal generation) or otherwise causing (e.g. electrical signal application) Plantar Flexion/Dorsiflexion. Plantar flexion and dorsiflexion are the movements involved when pointing the foot down and flexing it up, respectively. The gastrocnemius, soleus, tibialis posterior, fibularis brevis and longus, flexor hallucis longus, flexor digitorum longus and plantaris are the primary muscles acting in plantar flexion; and the tibialis anterior, extensor digitorum longus, extensor hallucis longus and peroneus tertius are primarily responsible for dorsiflexion. Further, Pronation/Supination is such that pronation occurs when the plantar side of the foot moves toward the floor surface in weight bearing, and supination occurs when the plantar side moves away from the floor surface. Pronation involves abduction, eversion and some dorsiflexion, whereas supination involves adduction, inversion and plantar flexion. As such, the electrical devices (i.e. the sensors 36 and/or actuators 38) can be used to detect (and cause) the muscle movement described by example only. As further discussed below, the EGM/EMS electrical signals are coordinated via operation of the control unit 40.

As one example operation, the garment 10 can used as be as a wearable sock providing automated muscle stimulation for diabetic foot neuropathy comprising in combination with the sock: stretch sensors 34; EMG electrodes 36 measuring the calf and plantarflexor muscles; EMS actuators 38 for actuating dorsiflexor muscles and plantarflexor muscles; IMU and Altimeter sensors 32 located on each sock for detecting steps, cadence and calories burned during walking, running, cycling and other exercises via IMU signal data collected. It is recognized that the EMG/EMS sensors 36,38 can be operated by the processor 116 to adaptively adjust muscle stimulation for foot neuropathy or similar applications.

### Controller Device 40

For example, the control unit 40, as further described below, can be responsible for receiving electrical EMG signals generated from the sensor 36 as well as supply (i.e. transmit) electrical EMS signals to the sensor 38. It is recognized that the controller device 40 can be decoupled rom the housing 124 for ease of cleaning of the garment 10, i.e. the controller device 40 can be releasably secured to the conductive pathway 42 network via the housing 124 coordinating electrical connection between the controller device 40 and the conducive pathways 42.

For example, the same sensor 36, 38 can be used to both generate and receive electrical signals, as desired. Alternatively, a different sensor 36 can be used to generate electrical EMG signals and a sensor 38 can be used to receive electrical EMS signals, as desired. The conductive pathways 42 are used to electrically couple the electrical components (e.g. sensors 36, 38) with the control unit 40. The conductive pathways 42 can comprise conductive wires or fibres applied to the body 11 of the garment 10. The conductive pathways 42 can comprise conductive fibres interlaced with the non-conductive interlaced fibres of the material of the body 11 of the garment 10. The control unit 40 can be one or more control units 40, as desired. The control unit 40 can be mounted to the wearer, for example directly to the body 11 of the garment 10. Alternatively, the control unit 40 can be positioned off the garment 10 and thus connected to the garment 10 via electrical conductors (e.g. wires, fibres) external to the garment 10.

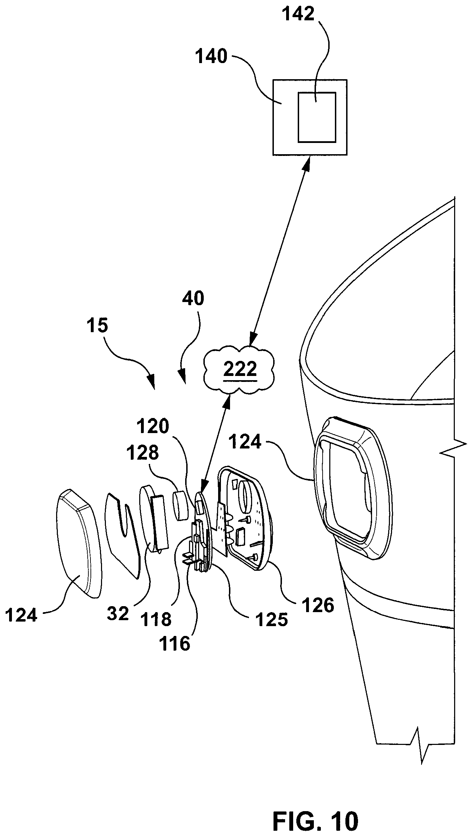

Also as described below, are electrical signal data (e.g. EMG) collected (i.e. representative of EMG generated by the body of the wearer 8 via the sensors 36 of the sensor platform - e.g. collection of sensors 32, 34, 36, 38, 39, 41, 43) and electrical signal data (e.g. EMG) expressed, i.e. representative of EMS received by the actuators 38 for subsequent processing by the actuators 38. Accordingly, the signal data expressed by the sensors 34, 36, 38, 39, 41,43 can be collected by the computing device 40 (see Figure 10) and processed by the computing device 40 to guide the subsequent generation of the signal data for consumption by the sensors/actuators 32, 34, 36, 38, 39, 41,43 of the sensor platform. For example, the signal data can be received by (or otherwise generated by) the computing device 40 as one or more commands for sending to the sensors/actuators 32, 34, 36, 38, 39, 41,43 (for subsequent processing thereby) of the sensor platform of the wearer 8.

As further described below, one example of the sensor platform is where temperature sensors 39 provide the signal data (e.g. output signals of the sensor platform) and heating elements as heating actuators 39 process the received signal data (e.g. as inputs to the sensor platform). For example, a garment 10 that can generate heat for wearers 8 that feel cold or need a skin contact based heating unit (e.g. actuator 39). The textile integrated temperature sensor 39 can monitor the wearer's 8 temperature and feedback that as signal data to the computing device 40 (see Figure 10), which can regulate the introduction of heat to the garment 10 via the heat actuators 39, similar to a thermostat. In this case, operation of the sensor platform can be customized and tuned to the personal requirements of each wearer 8, providing temperature profiles that are personalized and work per qualitative sensory requirements.

As further described below, one example of the sensor platform is where EMG sensors 36 provide the signal data (e.g. output signals of the sensor platform) and EMS elements as EMS actuators 38 process the received signal data (e.g. as inputs to the sensor platform). For example, a garment 10 that can generate EMG for wearers 8 need a skin contact based EMG unit (e.g. EMG sensor 38). The textile integrated EMG sensor 38 can monitor the wearer's 8 muscle activity and feedback that as signal data to the computing device 40 (see Figure 10), which can regulate the introduction of electrical stimulation of EMS signals to the garment 10 via the EMS actuators 38. In this case, operation of the sensor platform can be customized and tuned to the personal requirements of each wearer 8, providing muscle activity profiles that are personalized and work per qualitative sensory requirements.

As further described below, one example of the sensor platform is where pressure sensors 41 provide the signal data (e.g. output signals of the sensor platform) and pressure elements as pressure actuators 41 can process the received signal data (e.g. as inputs to the sensor platform). For example, a garment 10 that can generate pressure signals for wearers 8 need a skin contact based pressure unit (e.g. pressure sensor 41). The textile integrated pressure sensor 41 can monitor the wearer's 8 pressure activity and feedback that as signal data to the computing device 40 (see Figure 10), which can regulate the introduction of electrical stimulation signals to the garment 10 via the appropriate actuators. In this case, operation of the sensor platform can be customized and tuned to the personal requirements of each wearer 8, providing pressure activity profiles that are personalized and work per qualitative sensory requirements. It is recognized that the computing device 40 can control the operation of the sensor platform as a stand-alone unit. Alternatively, the computing device 40 can be in communication (via the communications network 222) with one or more networked devices 140 (see Figure 10), each running their respective applications for interpreting the signal data (e.g. received from the computing device 40 as sourced from the sensor platform) and for providing (e.g. to the computing device 40 for subsequent operation of the sensors/actuators 32, 34, 36, 38, 39, 41, 43 using the signal data) the signal data for expression by the sensor platform in response. In any case, it should be recognized that the sensor platform containing the sensors/actuators 32, 34, 36, 38, 39, 41, 43) operates as a textile based sensor platform in a bidirectional manner, i.e. generates the signal data and consumes the signal data.

As further described below, the signal data can be collected from the wearer 8 using the sensor platform (e.g. IMU, EMG, strain readings, temperature readings, pressure readings etc.) and can also be applied to the wearer 8 (generating heat, generating vibration, generating pressure, generating stimulation, etc. for application to the skin/body of the wearer 8) based on the signal data received by the wearer 8 (via and processed by the garment computer device 40).

For example, the wearer 8 can instruct the computer device 40 (or paired device 140) to generate one or more commands (see Figure 10) containing signal data collected as output of the wearer 8 and sent (e.g. over the network 222) as a signal input data to a corresponding sensor 34,36,38,39,41,43 of sensor platform of the user 8. For example, the computer device 40 can instruct (via a set of programmed instructions stored in memory) the sensor platform by generating one or more commands (see Figure 10) containing data as signal output and sent as signal input to a corresponding sensor 34,36,38,39,41,43 of the sensor platform of the wearer 8.

Referring again to Figure 10, optionally positioned on the garment 10 or otherwise coupled thereto, for example on an exterior surface 13 (i.e. outward facing from the wearer 8), is series of electrical components 15 including the computer device 40 including a computer processor 116, a memory 118 for executing stored instructions for receiving and processing of data obtained from the sensors 32,34,36,38,39,41,43, as well as communicating via a network interface 120 with the network 222 (e.g. Wi-Fi, Bluetooth, attached wired cable, etc.) as well as sending and receiving electrical signals from the sensors 32,34,36,38,39,41,43. The processor 116, memory 118 and network interface 120 can be mounted on a printed circuit board 126, which is housed in a housing 124 attached to the garment 10. Also connected to the PCB 125 can be the IMU sensor 32 for measuring the motion activity of the wearer 8. Also mounted in the housing 124 is a power supply 128 (e.g. battery) for powering the various electrical components 15 within the housing 124 as well as the sensors 32,34,36,38,39,41,43 external to the housing 24, connected via conductive communication pathways 42 (e.g. wires - see Figure 1 - woven into the fabric weave/knit of the garment 10 textile). The pathways 42 can be coupled to the sensors 32,34,36,38,39,41,43 via use of a conductive grommet, as desired.

Referring again to Figures 10 and others, the processor 116 (acting on stored 118 instructions) can transmit the collected signal data (in raw format and/or in preprocessed format from the sensors 32,34,36,38,39,41,43) to an external computer device 40 (e.g. smartphone or other desktop application) for viewing and/or further processing of the signal data. For example, the device 140 application can display the sensed data in a dashboard type format on a display 142 (or other type of GUI interface) for viewing by the wearer (or by another person other than the wearer that has been provided access to the data).

It is recognized that multiple sources of sensed data (e.g. temperature sensor 39 with activity/motion sensors 32 can be used in an algorithm stored in memory 118 to calculate various parameters of wearer 8 activity as desired). It is also realized that combinations of signal data can be used by the computer processor 116 to determine exercise activity being performed by the wearer, based on computer models of activity with typical sensor data.

As shown in Figure 10, the data processing system of the electrical components 15 can includes the central processing unit (CPU) 116, and a non-volatile memory storage device (DISC) 118 (such as a magnetic disc memory or electronic memory) and a read/write memory (RAM) 118 both in communication with the CPU 116. The DISC 118 includes data which, when loaded into the RAM 118, comprise processor instructions for the CPU 116 which define memory objects for allowing the device 40 to operate the applications(s) executing the functioning of the sensors/actuators 32,34,35,38,39,41,43. In view of the above descriptions of storage 118, the storage 118 can be configured as keeping the stored data (e.g. models and related data) in order and the principal (or only) operations on the stored data are the addition of and removal of the stored data from the storage (e.g. FIFO, FIAO, etc.). For example, the storage 118 can be a linear data structure for containing and subsequent accessing of the stored data and/or can be a non-linear data structure for containing and subsequent accessing of the stored data (e.g. models, associated model data such as features, effects, etc., signal data, applications, etc.). Further, the storage 118 receives various entities such as applicable data/instructions that are stored and held to be processed later. In these contexts, the storage 118 can perform the function of a buffer, which is a region of memory used to temporarily hold data while it is being moved from one place to another. Typically, the data is stored in the memory when moving the data between processes within/between one or more computers. It is recognized that the storage 118 can be implemented in hardware, software, or a combination thereof. The storage 118 is used in the system when there is a difference between the rate/time at which data is received and the rate/time at which the data can be processed.

### Example Applications of the sensor platform and controller device 40

An embodiment is described herein to effect muscle stimulation (any method is not part of the claimed invention). The present invention can provide automated muscle stimulation for diabetic foot neuropathy and also for other rehabilitation purposes. The present invention can provide on-demand muscle stimulation for the feet and the calf muscle, when the garment 10 is worn as a sock. The example operation combines stretch sensors 34 and IMU sensor 32 signals with EMG/EMS sensors 36,38 to adaptively (e.g. iteratively) adjust muscle stimulation for foot neuropathy or similar applications.

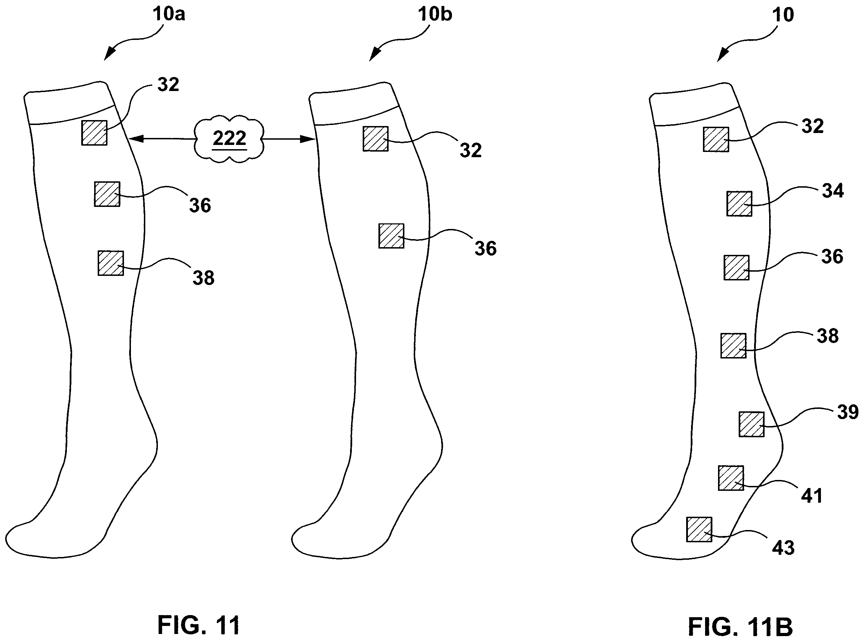

The embodiment is constructed from: EMG electrodes 36, EMS actuators 38, and IMU sensors 32. For example, the EMG electrodes 36 can measure EMG of the calf and plantar flexor muscles. The same electrodes 36 can be used for EMS actuation of the calf muscle, for example. EMS actuators 38 for dorsiflexor muscles and plantarflexor muscles can also be stimulated based on the sensor signal input of the sensors 32,36. It is recognized that the IMU and altimeter sensors 32 located on each sock 10 can be used for detecting steps, cadence and calories burned during walking, running, cycling and other exercises performed by the wearer 8. It is also recognized that the garment 10 can be provided as a pair of garments 10a, b, see Figure 11, such that signal data collected from the IMU 32 (and/or EMG sensors 36) of one garment 10a can be used as signal input for the controller device 40 of garment 10b (e.g. as communicated by example via the network interface 120 - see Figure 10, over the communications network 222), in order to generate signal data for driving of the EMS actuators 38 of the garment 10b. In this manner, the deemed normal operation of a normal/healthy foot (wearing garment 10a) can be used as a model for stimulating the activity of the deemed injured or unhealthy foot wearing the garment 10b. It is also recognized that a statistical model of the physical activity of the deemed heathy foot (e.g. worn by garment 10a) can be recorded and then stored in the controller device 40 of garment 10b. As such, the controller device 40 of garment 10b can use the statistical model (e.g. containing sensor 32,34,36,39,41 signal data collected over operational activity of the deemed heathy/normal foot of the wearer 8), stored as representative of desired stimulation (i.e. generated signal data) needed for operation of the sensor platform of sensors 32,34,36,38,39,41,43 of the garment 10b.

A method for monitoring EMG for the calf muscle is presented. In rehabilitation of injuries caused to the calf muscle, such as a tear, the method measures EMG signal data periodically during the day in the stationary phases. Features can be extracted from the EMG signal data and compared with the features from the previous EMG test and also with the normal standard. The improvement in condition can be estimated to give feedback to the wearer 8 for adjustment of therapy and other rehabilitation measures. In some embodiments, the EMG signal will be divided by the processor 116 into a plurality of segments or frames for analysis. In some embodiments, the raw the EMG signal can be used by the processor 116 with a stored neural network type machine learning algorithm to detect changes by the processor 116 in the EMG signal data during the monitoring process by the processor 116.

In some embodiments, the EMG signal can be preprocessed by the processor 116 using low- and high-pass filters to remove DC offset and high frequency noise. The features extracted from the EMG signal data, raw and processed, can include statistical measures such as mean, standard deviation, range, number of zero-crossings, time interval between zero-crossings, root mean square energy and power, and mean absolute standard deviation. The features from frequency domain can consist of fundamental frequency in the frame under consideration, power of the fundamental frequency, power in frequency sub-bands and spectral entropy measures. The features can also be extracted by the processor 116 from the joint time-frequency domain using wavelet transformations, short-time Fourier transform.

The features can be used to train a machine learning classifier such as, but not limited to, linear or logistic regression, neural networks, support vector machine to provide continuous and categorical output regarding the condition of the calf muscle as represented in the statistical model as described above.

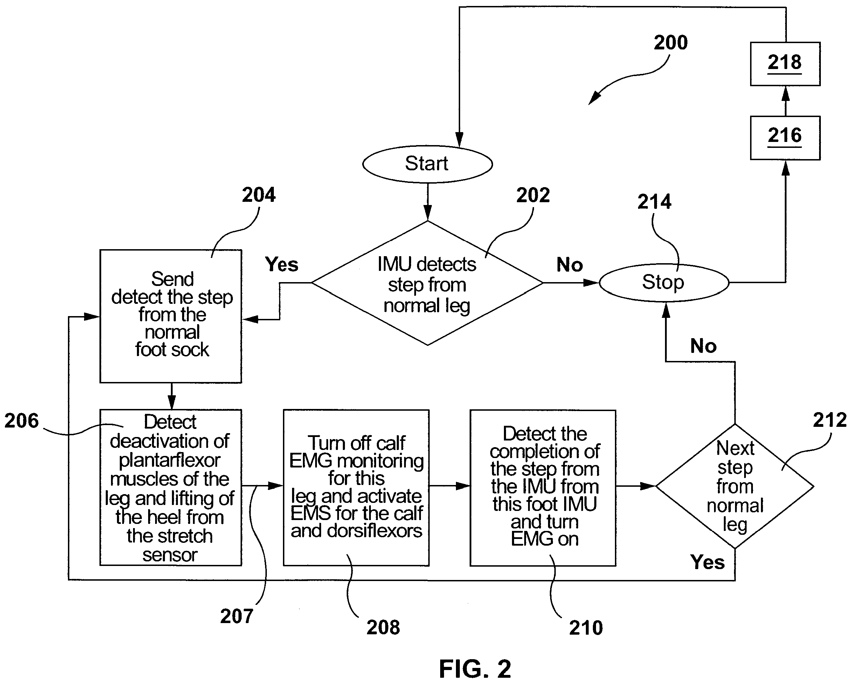

An example method is presented in Figure 2 for monitoring EMG for the calf muscle during rehabilitation and providing EMS to the calf muscle periodically during the day for the wearer 8 of the garment 10 as one or more socks. The EMS is adjusted by the processor 116 based on improvement of the muscle condition from EMG signal data collected by the processor 116. The EMG features and algorithm can be used by the processor 116 for monitoring the muscle condition and based on output of the EMG algorithm executed by the processor 116, the EMS feedback generated by the processor 116 can be adjusted to stimulation to the calf muscle by the EMS actuators 38 in the recovery or rehabilitation phases of the wearer 8.

In some embodiments of the socks, EMS electrodes 38 can be placed on the sole region (as shown in Fig. 1) of the sock to provide additional EMS for the plantar and heel region.

The example method 200 presented can be used for aiding in walking during foot drop condition caused by diabetes or multiple sclerosis. For the specific application, one sock 10a (for the normal leg and foot) can contain the IMU sensor 32 while the other sock 10b can contain EMG 36, EMS 38 and stretch 34 sensors. The signals from all the sensors on both socks 10a,b are provided as input/output signal data together for the main algorithm as executed by the processor 116 of the controller device 40 of the sock 10b. As shown in Figure 2, the IMU sensor 32 can detect walking from the normal leg (using sock 10a) and switch the EMG 36 and EMS 38 on and off on the other leg (using sock 10b) to aid the subject to walk properly.

At step 202, the IMU signal from the normal sock 10a can be used to detect the first step using the time-domain accelerometer signal data from the normal leg 10a IMU sensor 32, and this signal data is sent 204 to the controller device 40 of sock 10b. The EMG sensors 36 on the other leg (sock 10b) will be turned on at step 206 to monitor activation of plantarflexor muscles of the foot wearing sock 10b. As the step/activity from the normal leg reaches completion and the subject starts to move his other leg wearing sock 10b (for example as detected 207 by the IMU sensor 32 of sock 10b as received by the processor 116 of the controller device 40 of sock 10b), the algorithm as implemented by the processor 116 can detect this next step, e.g. from the IMU signal from the sock 10a,b, and/or via deactivation of the plantarflexor muscles as the EMG signal data of the sock 10b will drop at step 208 below a certain threshold. This can turn the EMG mode off by the processor 116 (e.g. deactivate the EMG sensors 36 signal data collection) and turn on the EMS electrodes 38 by the processor 116 on the dorsiflexors and also the calf muscles to provide stimulation and aid in lifting the foot of sock 10b properly by the wearer 8. As the step of sock 10b reaches its completion as detected by IMU sensor 32 by the processor 116 of sock 10b, at step 110, the EMS actuators 38 can be turned off by the processor 116 and EMG sensors 36 can be turned on again by the processor 116. At step 212, the process repeats if the IMU sensor 32 data from the sock 10a determines that the next step is being taken by the sock 10a of the deemed healthy foot. Otherwise, the method stops at step 214.

As discussed, the method 200 for monitoring EMG for the calf muscle can comprise one or more of the following activities: dividing the EMG signal into segments or frames for analysis; the raw EMG signal can be used with a neural network type machine learning algorithm by the processor 116 to detect changes in the EMG signal during the monitoring process; the EMG signal can be preprocessed by the processor 116 using low- and high-pass filters to remove DC offset and higher frequency noise; the features extracted from the EMG signal by the processor 116, raw and/or processed, can include statistical measures such as mean, standard deviation, range, number of zero-crossings, time interval between zero-crossings, root mean square energy and power, and mean absolute standard deviation; the features from frequency domain can consist of fundamental frequency in the frame under consideration, power of the fundamental frequency, power in frequency sub-bands and spectral entropy measures; the features can also be extracted by the processor 116 from the joint time-frequency domain using wavelet transformations, short-time Fourier transform; and/or the features can be used to by the processor 116 train a machine learning classifier such as, but not limited to, linear or logistic regression, neural networks, support vector machine to provide continuous and/or categorical output regarding the condition of the (e.g. calf) muscle.

As discussed above, the method 200 for monitoring EMG for the (e.g. calf) muscle during rehabilitation and providing EMS to the muscle periodically during the day can comprise the following activities: the EMS stimulation degree can be adjusted by the processor 116 based on improvement of the muscle condition from EMG sensor 36 signal data readings as interpreted by the processor 116; the EMG features and algorithm can be used for monitoring the muscle condition by the processor 116; and/or based on output of the EMG algorithm, the EMS feedback can be adjusted by the processor 116 to stimulate the muscle in the recovery or rehabilitation phases.

As such, the monitoring of the sensors 36,38can be used to detect and stimulate movement of the unhealthy foot wearing the sock 10b. In some embodiments, a threshold comparison method can be used to detect at steps 208, 210 activation and deactivation of the plantar flexors, e.g. sensor signals 32,34,36,38 compared to the stored threshold(s) and determined as matching (e.g. above or below) a stated stored (in memory 118) threshold can be deemed by the processor 116 to be representative of a step being started and/or finished.

In some embodiments, statistical features can be extracted from the EMG signal data to detect the activation and deactivation of plantar flexors. If the subject remains stationary in sitting or lying positions for a certain duration of time, the EMG electrodes 36 can be turned off on the sock 10 to avoid battery usage. This can be shown by monitoring at step 216 for activity of the sensors 32,34,36,38, and if not activity is detected (e.g. by any of the signal generation activities in steps 202-212) after a specified period of time, at step 218 the EMG sensors 36 are deactivated until step 202 is again started.

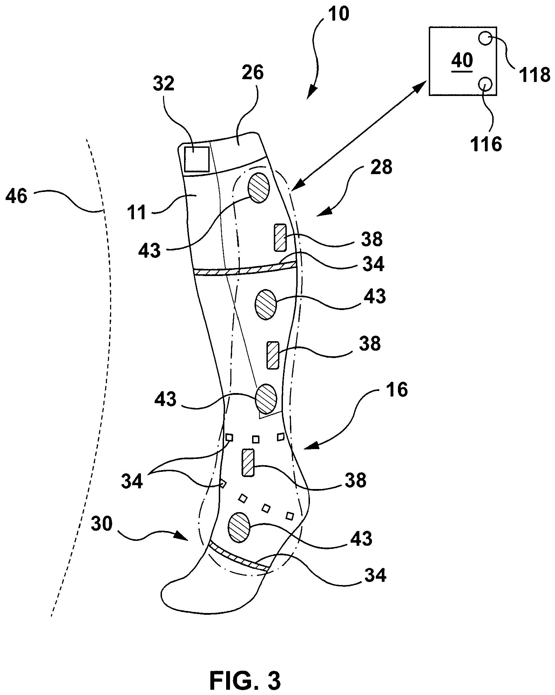

Referring to Figure 3, shown is an example of the garment 10 having bio impedance sensors 43, where bio impedance sensors 43 provide the signal data (e.g. output signals of the sensor platform) and bio impedance elements as can generate the signal data (e.g. as inputs to the sensor platform). For example, a garment 10 that can generate bio impedance signals for wearers 8 need a skin contact based bio impedance unit (e.g. bio impedance sensor 43). The textile integrated bio impedance sensor 43 can monitor the wearer's 8 bio impedance over time and feedback that as signal data to the computing device 40 (see Figure 10), which can regulate the introduction of electrical stimulation signals to the garment 10 via the appropriate actuators 38. In this case, operation of the sensor platform can be customized and tuned to the personal requirements of each wearer 8, providing bio impedance activity profiles that are personalized and work per qualitative sensory requirements.

Combining Bio-Impedance 43 and Stretch 34 Sensing for multiple functions. Combined bioimpedance 43 and stretch 34 sensors can be used for edema detection and similar ankle joint swelling. Combine bioimpedance sensors 43 and EMS actuators 38 can be used for improvement of blood flow in the lower leg and foot for healing of ulcers. The bio impedance sensors 43 are positioned along the body 11 of the garment 10, in particular in one or more locations of the top portion 28 and bottom portion 30. Further, in the described combination of sensors 34,43, a plurality of the stretch/strain sensors 34 can be positioned at various individual locations spaced apart along a longitudinal axis 46 of the garment 10. For, example, a pair of sensors 34 can be positioned adjacent to and on either side of the intermediate region 16. Alternatively or in addition to, a pair of sensors 34 can be positioned away from and on either side of the intermediate region 16 in the top portion 28 and the bottom portion 30. It is recognized that bio impedance sensors 43 are used to measure fluid content in the limb tissues, as a current is passed between pairs of bio impedance sensors 43 through the limb tissue, as controlled by the processor 116. The processor 116 interprets the current measurements as calibrated against a set of fluid content values stored in the storage 118 (e.g. bio impedance is about the electrical properties of the body, e.g. to what extent the body is a good conductor, such that bio impedance is a measure of how well the body impedes electric current flow - recognizing fat has high resistivity while blood/fluid has lower relative resistivity). Accordingly, as the resistivity in the limb goes down, the processor 116 would determine that the fluid content (i.e. swelling) of the limb is rising. Further, increases in strain/stretch by the stretch sensors 34 signal data can also, or in addition to the bio impedance signal data, be interpreted by the processor 116 as indicative of swelling increase or decrease over time.

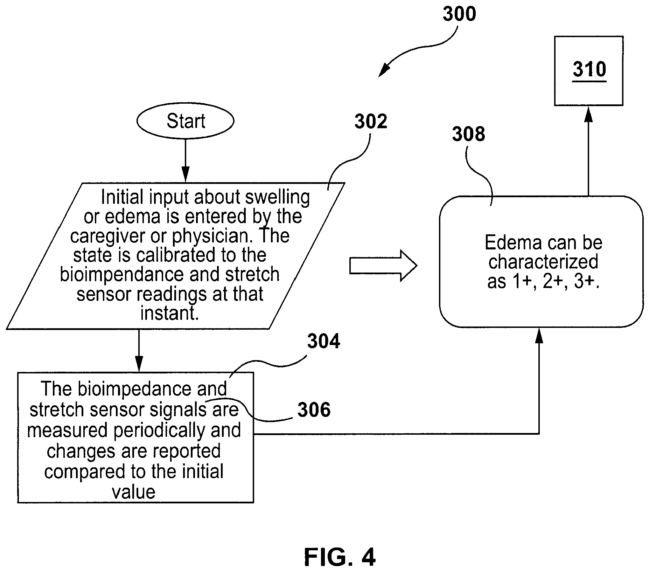

A method 300 of Figure 4 is presented for estimation of fluid content in the limb tissues for detection of peripheral swelling and edema of the limb using the combination of sensors 34,43 shown in the garment 10 embodiment of Figure 3. An adaptive algorithm stored in memory 118 is executed by the processor 116 at step 302 to store in memory 118 sensor 43 and sensor 34 readings as indicative of a swelling state for a selected time/day. At step 304, bio impedance sensors 43 are used by the processor 116 by passing current through the limb between selected pairs of bio impedance sensors 43. Separately at step 306, the processor reads signal data generated by the strain sensors 34 to determine degree of stretch 34 at the locations of the strain sensors 34. It is recognized that the sensor 43 readings and the sensor 34 readings must be done sequentially, in order to inhibit interference between the sensor readings 43,34. At step 308, the processor 116 compares the sensor 34,43 signal data collected with the stored values in the memory 118 in order to determine whether the swelling has increased, stayed the same, or decreased. At step 310, the determined swelling findings are reported to the wearer 8 and/or the wearer's physician in order to track changes in fluid content and swelling of the limb on periodic basis.

In some embodiments, the algorithm implemented by the processor 116 can be trained on absolute values from bioimpedance 43 and stretch 34 sensors can be combined with a linear regression or threshold detector or some non-linear estimator to detect increase in fluid content of the leg tissue. In some embodiments, the increase or decrease in swelling will be determined from the change in bioimpedance 43 and stretch 34 sensor readings compared with the initial calibration or reading from the first time use. The change in values can be used by the processor 116 to estimate the increase or decrease in edema or swelling in the foot.

In some embodiments, the methods presented above can be used with any textile garment 10 tailored in the form of but not limited to a sleeve, glove etc., with bio impedance 43 and stretch 34 sensors for either absolute measurement or increase and decrease of swelling or edema at a particular location on the body of the wearer 8.

Referring again to Figure 3, the EMS actuators 38 present can be used by the processor 116 to increase blood circulation in the limb and thus help to decrease swelling in the limb. Accordingly, in the event that the processor detects swelling as per the method 300, the processor 116 can selectively activate the EMS actuators 38 of the garment 10 to promote/increase circulation in an effort to decrease the detected swelling. Alternatively, the method 400 can be used for facilitating healing of wounds and ulcers.

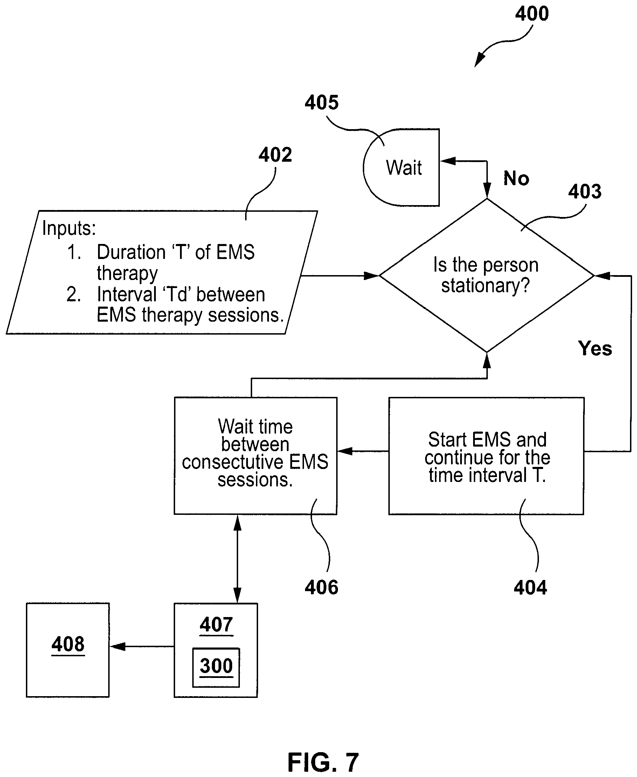

For example, the method (see Figure 7) 400 is presented to increase blood circulation in the limb (e.g. leg and foot region) using EMS actuators 38. The time duration T for EMS therapy, degree of stimulation, and period Td between consecutive therapy sessions can be input through a software application stored in memory 118, as implemented by the processor 116. At step 402, the duration time T of the EMS therapy, degree of stimulation, and an interval between therapy sessions is input to the memory 118. Based on these values, the EMS therapy (activation of the EMS actuators 38 by the processor 116) will start at step 404 whenever the person is detected as being stationary at step 403 based on signal data from the IMU sensor 32 received by the processor 116. At step 406, once the stimulation of time interval T is completed, the processor 116 can wait (i.e. maintain the EMS actuators 38 as inactive) for the specified in wait interval Td before continuing at step 404 based on the parameters specified as input. It is recognized that between the sessions of duration T, the processor 116 can check iteratively at step 403 to confirm that the wearer 8 is stationary before proceeding at step 404, otherwise the processor 116 waits at step 405 before trying again at step 403. The IMU sensor 32 present on the garment 10 (as shown in Fig. 3) can be used to detect rest and movement periods of the wearer 8. It is also recognized that during the wait period at step 406, the processor 116 can perform method 300 (see Figure 4) in order to reevaluate the swelling of the limb at step 407. In the event that the limb is deemed to have sufficiently reduced swelling, the processor 116 can stop the EMS stimulation at step 408 or otherwise continue at step 403.

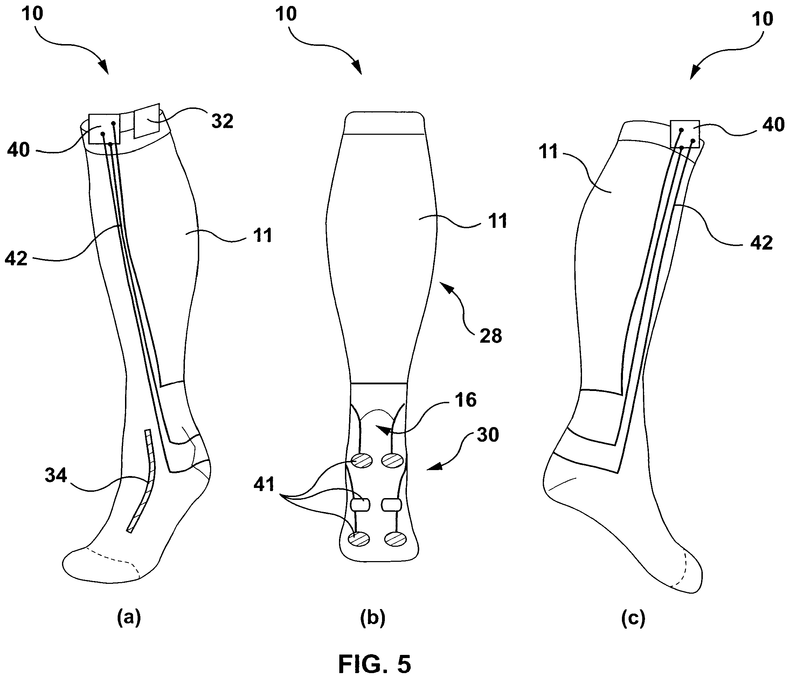

Referring to Figure 5, shown are a plurality of pressure sensors 41 positioned on the (e.g. sole) of the garment 10, such as on the rear region 20 and on the bottom portion 30 (see Figure 8). The pressure sensors 41 can be positioned in pairs along the body 11 as spaced between the second end 14 and the intermediate region 16. In some embodiments, the controller device 40 can use a combination of sole-located pressure sensors 41, stretch sensors 34 and IMU sensors 32 for posture, gait and balance monitoring, as desired.

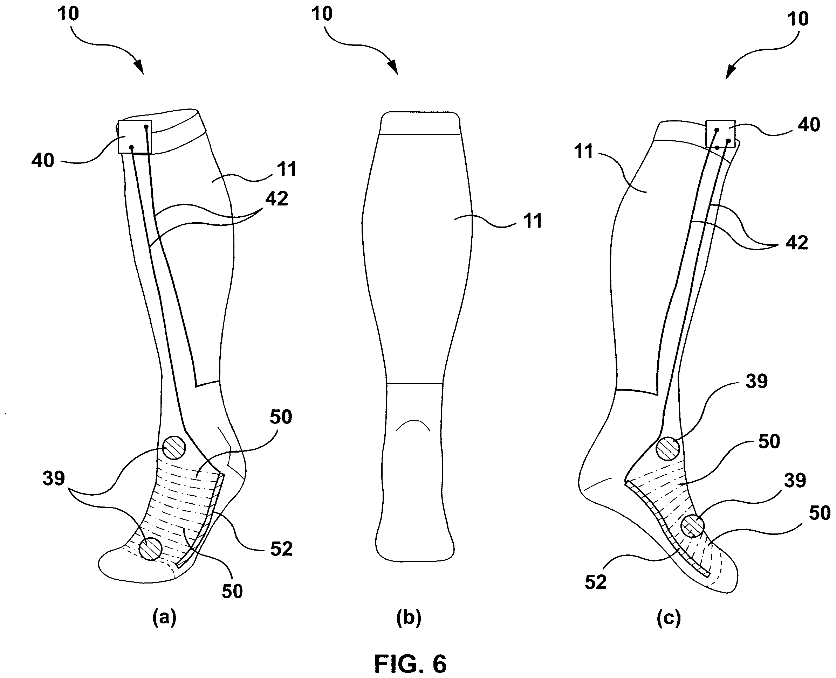

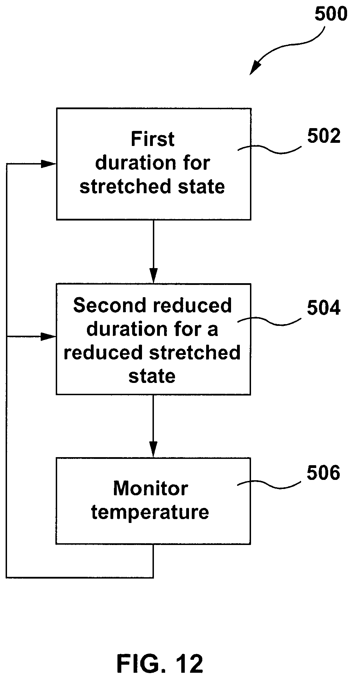

Referring to Figure 6, in yet another embodiment, the garment 10 can incorporate measuring temperature and providing heat to the body 11. A method to measure and control temperature using adaptive power regulation to compensate for the change in resistance when knitted heating elements 50 are stretched can be implemented. The heating elements 50 can be conductive fibres spaced apart on/in the body 11 along a portion of the body 11, e.g. in the lower region 30 (see Figure 8). Each of the resistive elements is connected in parallel to a pair of power buses 52 connected on either end of the resistive elements 50, being in parallel. The power buses 50 are connected to the controller device 40 (and thus the power supply and processor 116) via the conductive pathways 42. Also provided can be separate temperature sensors 39 also coupled to the controller device 40 in order to measure localized temperature of the body 11 at one or more distributed locations. The processor 116 can be programmed via stored instructions in memory 118 in order to implement a temperature control method 500 (see Figure 12).

In normal resistive heating applications, a voltage, V0, is applied across an electrically resistive element, R0 (e.g. element fibre 50), producing a current, I0. The resulting power, P0, can be expressed as I0V0, or V02/R0 or I02xR0. The challenge when providing constant temperature to wearable garments10 is that the resistive element 50 is typically conductive yarns or fibers which can change resistance significantly during activity of the wearer 8, i.e., the electrical resistance of each of the resistive element 50 increases when the fabric body 11 is stretched. Furthermore, the resistance of each resistive element 50 also typically increases for most conductive materials (e.g. silver, copper, etc...) when the temperature of the resistive element 50 increases due to thermal coefficient of resistance. Constant current circuitry and adding temperature sensors 39 can be too complex and bulky to add to garments 10. Therefore a novel method 500 of temperature regulation of the resistive elements 50 is provided, involving controlling the power applied to the resistive elements 50 by the processor 116 through time-domain pulse width regulation is employed.

A typical example would be a 12V DC power source 128 (see Figure 10) applied to a knitted conductive yarn resistive heating element(s) 50 having a resistance change range from 1.2-ohm (un-stretched yarn) to 12- 10 ohms (fully stretched). The desired power can be 12W, which it can be seen is achieved in the fully stretched position. I = V/R = 12V/12-ohm = 1A. Power = IxV = 12V x A = 12W. From experimentation, it was determined that when this power is pulsed ON for 10 sec and OFF for 1 sec (e.g. employing a time domain pulse width modulation/regulation), then a temperature of 25C could be maintained on the garment 10. Total energy (E) delivered in this heating cycle = 12W x 10 sec = 120 joules. However, when the yarn returns to the un-stretched position, continuing as I = V/R = 12V/1.2-ohm = 10A. Power = IxV = 10A x 12V = 120W can create too high of a temperature. Hence, the solution employed by the method 500 is to use pulse-width-modulation (PWM) in the time domain, such that the ratio of the time that the power is turned ON for (e.g. only 1 second) to maintain the same (e.g. 25C) temperature on the garment 10. The duration of time of the un-stretched yarn power pulse corresponds to a fraction (e.g. 1/10^{th}) of the duration of time of the fully-stretched yarn power pulse, which results in a reduction (e.g. 1/10^{th}) of the total energy (E) delivered to the garment 10 via the resistive element(s) 50. Total energy (E) delivered in this heating cycle = 120W x 1 sec = 120 joules, by example only.

It can be appreciated that this time-domain based pulse-width-modulated temperature control method 500 can be applied to resistive heating element 50 based system where the resistance of the heating element is changing. Thus, the controller device 40 is configured via stored instructions, when executed by the processor 116 to incorporate measuring temperature and providing heat to the garment 10 using a method to measure and control temperature using adaptive power regulation to compensate for the change in resistance when knitted heating elements 50 are stretched. Referring to Figure 12, at step 502, the processor 116 applies a current for a first duration of time and then off in a pulsed application mode to one or more resistive elements 50 of the garment 10 when the resistive element(s) 50 are in a first stretched state in order to increase a temperature of the body 11 of the garment 10. At step 504, the processor 116 applies a current for a fraction of the first duration of time then off in using the pulsed application mode to the one or more resistive elements 50 of the garment 10 when the resistive element(s) 50 are in a second stretched state, such that the degree of stretch in the second stretched state is less than the degree of stretch in the first stretched state. The processor 116 can also monitor 506 the temperature of the body 11 using the temperature sensor(s) 39 during the pulsed application mode, recognizing that for an increased temperature detected over a selected threshold (e.g. set temperature), the processor can decrease the duration of the applied current during the ON phase of the pulse-width-modulation (PWM) in the time domain applied by the processor 116, in order to maintain the temperature of the body 11 of the garment 10.

Thus, it is appreciated that the optimum dimensional relationships for the parts of the invention, to include variation in size, materials, shape, form, function, and manner of operation, assembly and use, are deemed readily apparent and obvious to one of ordinary skill in the art, and all equivalent relationships to those illustrated in the drawings and described in the above description are intended to be encompassed by the present invention. For example, in reference to Figure 11B, it is recognized that any of the garments 10 can include one or more of the sensor types 32,34,36,38,39,41,43, in any combination of sensor types as desired (i.e. one type, many types selected from the types but not all types, or all types).

Furthermore, other areas of art may benefit from this method and adjustments to the design are anticipated. Thus, the scope of the invention should be determined by the appended claims, rather than by the examples given.

## Claims

1. A tubular garment (10) comprising a controller device (40) the tubular garment comprising a plurality of interlaced non-conductive fibres making up a body (11) of the garment including:
a top portion (28) and a bottom portion (30) of the body separated by an intermediate portion (16), the intermediate portion for positioning over a joint of limb of a wearer of the garment;
a network of conductive pathways (42) in the body for connecting to the controller device:
a strain sensor (34) of the body positioned across the intermediate portion and coupled to the network of conductive pathways;
an inertial measurement unit (IMU) sensor (32) mounted on the body and configured for communication with the controller device;
a plurality of sensors (36) of the body for providing electromyography (EMG) functionality and a plurality of actuators (38) for providing electrical muscle stimulation (EMS) functionality with respect to one or more muscles of the wearer positioned adjacent to the body when the garment is worn by the wearer, the plurality of sensors and the plurality of actuators connected to the network of conductive pathways;
wherein the controller device is programed to operate the EMG and EMS functionality based on signal data obtained from the IMU sensor.

2. The garment of claim 1, wherein the joint is an ankle, the limb is a leg and the body forms a sock having an open end and a closed end.

3. The garment of claim 1, wherein at least one of the strain sensor and the plurality of sensors comprise conductive fibres interlaced with the nonconductive fibres making up the body.

4. The garment of claim 2, wherein the plurality of sensors are positioned in a rear portion of the top portion and to either side of the top portion, the top portion being adjacent to the open end.

5. The garment of claim 4, wherein the plurality of sensors positioned in the rear portion are positioned in pairs to either side of a longitudinal centerline of the garment extending between the open end and the closed end.

6. The garment of claim 4 further comprising the plurality of sensors having sensors positioned in the rear portion of a bottom portion of the garment.

7. The garment of claim 1 further comprising the controller device configured to detect motion of the wearer via the signal data and to perform the EMG and EMS functionality sequentially.

8. The garment of claim 1 further comprising one or more bio impedance sensors of the body.

## Patentansprüche

1. Schlauchförmiges Kleidungsstück (10), das eine Steuerungsvorrichtung (40) umfasst, wobei das schlauchförmige Kleidungsstück mehrere verflochtene nichtleitfähige Fasern umfasst, die einen Körper (11) des Kleidungsstücks bilden, einschließlich:
eines oberen Teils (28) und eines unteren Teils (30) des Körpers, getrennt durch einen Zwischenteil (16), wobei der Zwischenteil zum Positionieren über einem Gelenk einer Gliedmaße eines Trägers des Kleidungsstücks dient;
ein Netz leitfähiger Pfade (42) in dem Körper zum Verbinden der Steuerungsvorrichtung;
einen Dehnungssensor (34) des Körpers, positioniert über dem Zwischenteil und gekoppelt mit dem Netz leitfähiger Pfade;
einen Sensor (32) einer inertialen Messeinheit (IMU), montiert an dem Körper und ausgelegt zur Kommunikation mit der Steuerungsvorrichtung;
mehrere Sensoren (36) des Körpers zum Bereitstellen einer Elektromyografie(EMG)-Funktionalität und mehrere Aktuatoren (38) zum Bereitstellen einer Elektromuskelstimulations(EMS)-Funktionalität mit Bezug auf einen oder mehrere Muskeln des Trägers, die an den Körper angrenzend positioniert sind, wenn das Kleidungsstück durch den Träger getragen wird, wobei die mehreren Sensoren und die mehreren Aktuatoren mit dem Netz leitfähiger Pfade verbunden sind;
wobei die Steuerungsvorrichtung dazu programmiert ist, die EMG- und EMS-Funktionalität basierend auf von dem IMU-Sensor erhaltenen Signaldaten zu betreiben.

2. Kleidungsstück nach Anspruch 1, wobei es sich bei dem Gelenk um einen Knöchel handelt, es sich bei der Gliedmaße um ein Bein handelt und der Körper eine Socke mit einem offenen Ende und einem geschlossenen Ende bildet.

3. Kleidungsstück nach Anspruch 1, wobei mindestens einer des Dehnungssensors und/oder die mehreren Sensoren leitfähige Fasern umfassen, die mit den den Körper bildenden nichtleitfähigen Fasern verflochten sind.

4. Kleidungsstück nach Anspruch 2, wobei die mehreren Sensoren in einem hinteren Teil des oberen Teils und an jeder Seite des oberen Teils positioniert sind, wobei der obere Teil an das offene Ende angrenzt.

5. Kleidungsstück nach Anspruch 4, wobei die mehreren in dem hinteren Teil positionierten Sensoren paarweise an jeder Seite einer Längsmittellinie des Kleidungsstücks, die sich zwischen dem offenen Ende und dem geschlossenen Ende erstreckt, positioniert sind.

6. Kleidungsstück nach Anspruch 4, das ferner umfasst, dass die mehreren Sensoren Sensoren aufweisen, die in dem hinteren Teil eines unteren Teils des Kleidungsstücks positioniert sind.

7. Kleidungsstück nach Anspruch 1, das ferner umfasst, dass die Steuerungsvorrichtung dazu ausgelegt ist, eine Bewegung des Trägers über die Signaldaten zu erkennen und die EMG- und EMS-Funktionalität nacheinander durchzuführen.

8. Kleidungsstück nach Anspruch 1, das ferner einen oder mehrere Bioimpedanzsensoren des Körpers umfasst.

## Revendications

1. Vêtement tubulaire (10) comprenant un dispositif de commande (40), le vêtement tubulaire comprenant une pluralité de fibres non conductrices entrelacées constituant un corps (11) du vêtement, comprenant :
une partie supérieure (28) et une partie inférieure (30) du corps séparées par une partie intermédiaire (16), la partie intermédiaire étant destinée au positionnement sur une articulation d'un membre d'un porteur du vêtement ;
un réseau de voies conductrices (42) dans le corps pour la connexion au dispositif de commande ;
un capteur de contrainte (34) du corps positionné à travers la partie intermédiaire et couplé au réseau de voies conductrices ;
un capteur d'unité de mesure inertielle (IMU) (32) monté sur le corps et configuré pour communiquer avec le dispositif de commande ;
une pluralité de capteurs (36) du corps pour fournir une fonctionnalité d'électromyographie (EMG) et une pluralité d'actionneurs (38) pour fournir une fonctionnalité de stimulation musculaire électrique (EMS) par rapport à un ou plusieurs muscles du porteur, positionnés de manière adjacente au corps lorsque le vêtement est porté par le porteur, la pluralité de capteurs et la pluralité d'actionneurs étant connectés au réseau de voies conductrices ;
le dispositif de commande étant programmé pour faire fonctionner les fonctionnalités EMG et EMS sur la base des données de signal obtenues à partir du capteur IMU.

2. Vêtement selon la revendication 1, l'articulation étant une cheville, le membre étant une jambe et le corps formant une chaussette ayant une extrémité ouverte et une extrémité fermée.

3. Vêtement selon la revendication 1, au moins un du capteur de contrainte et de la pluralité de capteurs comprenant des fibres conductrices entrelacées avec les fibres non conductrices qui constituent le corps.

4. Vêtement selon la revendication 2, la pluralité de capteurs étant positionnés dans une partie arrière de la partie supérieure et de chaque côté de la partie supérieure, la partie supérieure étant adjacente à l'extrémité ouverte.

5. Vêtement selon la revendication 4, la pluralité de capteurs positionnés dans la partie arrière étant positionnés par paires de part et d'autre d'une ligne centrale longitudinale du vêtement, s'étendant entre l'extrémité ouverte et l'extrémité fermée.

6. Vêtement selon la revendication 4, comprenant en outre la pluralité de capteurs ayant des capteurs positionnés dans la partie arrière d'une partie inférieure du vêtement.

7. Vêtement selon la revendication 1, comprenant en outre le dispositif de commande configuré pour détecter le mouvement du porteur par l'intermédiaire des données du signal et pour réaliser les fonctionnalités EMG et EMS de manière séquentielle.

8. Vêtement selon la revendication 1, comprenant en outre un ou plusieurs capteurs de bio-impédance du corps.
